# EUROPEAN PATENT SPECIFICATION

(11) **EP 3 377 080 B1**
(45) Date of publication and mention of the grant of the patent: **14.02.2024**
(21) Application number: 16867239.2
(22) Date of filing: 18.11.2016
(51) Int. Cl.: A61K 35/44, A61K 38/08, A61K 38/10, A61K 38/39, A61K 39/145, C07K 7/08, C07K 14/78, G01N 33/574

(54) **PEPTIDES WITH ANTI-ANGIOGENIC, ANTI-LYMPHANGIOGENIC, AND ANTI-EDEMIC PROPERTIES AND NANOPARTICLE FORMULATIONS**
PEPTIDE MIT ANTI-ANGIOGENEN, ANTI-LYMPHANGIOGENEN UND ANTI-ÖDEMATÖSEN EIGENSCHAFTEN UND NANOPARTIKELFORMULIERUNGEN
PEPTIDES À PROPRIÉTÉS ANTI-ANGIOGÉNIQUES, ANTI-LYMPHANGIOGÉNIQUES, ET ANTI- OEDÉMIQUES ET FORMULATIONS DE NANOPARTICULES

(30) Priority: 19.11.2015 US 201562257569 P
(43) Date of publication of application: 26.09.2018
(73) Proprietor: Asclepix Therapeutics, LLC, Baltimore, MD 21211 (US)
(72) Inventor: BRESSLER, Eric M., Baltimore, MD 21211 (US); GREEN, Jordan J., Baltimore, MD 21211 (US); PANDEY, Niranjan B., Baltimore, MD 21211 (US); POPEL, Aleksander S., Baltimore, MD 21211 (US); SHMUELI, Ron B., Baltimore, MD 21211 (US)
(74) Representative: Grund, Martin
(86) International application number: PCT/US2016/062816
(87) International publication number: WO 2017/087825

(56) References cited:
- WO-A1-2014/018018
- WO-A1-2014/197892
- WO-A1-2014/197892
- WO-A1-2015/051010
- WO-A2-2012/079088
- US-A1- 2014 100 164
- US-A1- 2014 256 614
- ESAK LEE ET AL: "Inhibition of breast cancer growth and metastasis by a biomimetic peptide", SCIENTIFIC REPORTS, vol. 4, 20 November 2014 (2014-11-20), page 7139, XP55296374, DOI: 10.1038/srep07139
- G. K. PHILIPS ET AL: "Therapeutic uses of anti-PD-1 and anti-PD-L1 antibodies", INTERNATIONAL IMMUNOLOGY, vol. 27, no. 1, 16 October 2014 (2014-10-16), pages 39-46, XP55217958, ISSN: 0953-8178, DOI: 10.1093/intimm/dxu095
- NORA GRAF ET AL: "[alpha] V &bgr; 3 Integrin-Targeted PLGA-PEG Nanoparticles for Enhanced Anti-tumor Efficacy of a Pt(IV) Prodrug", ACS NANO, vol. 6, no. 5, 22 May 2012 (2012-05-22), pages 4530-4539, XP55237224, US ISSN: 1936-0851, DOI: 10.1021/nn301148e
- GRAF, N ET AL.: 'Alpha]V[beta]3 Integrin-targeted PLGA-PEG nanoparticles for enhanced anti-tumor efficacy of a Pt(IV) prodrug.' ACS NANO. vol. 6, no. ISSUE, 14 May 2012, pages 4530 - 4539, XP055237224
- ARMSTRONG, SM ET AL.: 'Influenza Infects Lung Microvascular Endothelium Leading to Microvascular Leak: Role of apoptosis and Claudin-5.' PLOS ONE. vol. 7, no. 10, 24 October 2012, pages 1 - 14, XP055383196
- HELLMAN, J: 'Addressing the Complications of Ebola and Other Viral Hemorrhagic Fever Infections: Using Insights from Bacterial and Fungal Sepsis.' PLOS PATHOLOGY. vol. 11, no. 10, 01 October 2015, pages 1 - 9, XP055383201
- OTT, PA ET AL.: 'Inhibition of immune checkpoints and vascular endothelial growth factor as combination therapy for metastatic melanoma: an overview of rationale, preclinical evidence, and initial clinical data.' FRONTIERS IN ONCOLOGY vol. 5, no. 202, 22 September 2015, XP055383215
- FLORENCE SCHAFFNER ET AL: "Integrin [alpha]5[beta]1, the Fibronectin Receptor, as a Pertinent Therapeutic Target in Solid Tumors", CANCERS, vol. 5, no. 4, 15 January 2013 (2013-01-15), pages 27-47, XP055697383, DOI: 10.3390/cancers5010027
- YASUFUMI SATO: "Persistent vascular normalization as an alternative goal of anti-angiogenic cancer therapy", CANCER SCIENCE, vol. 102, no. 7, 14 April 2011 (2011-04-14), pages 1253-1256, XP055697390, JP ISSN: 1347-9032, DOI: 10.1111/j.1349-7006.2011.01929.x
- PARK JIN-SUNG ET AL: "Normalization of Tumor Vessels by Tie2 Activation and Ang2 Inhibition Enhances Drug Delivery and Produces a Favorable Tumor Microenvironment", CANCER CELL, CELL PRESS, US, vol. 30, no. 6, 12 December 2016 (2016-12-12), pages 953-967, XP029845233, ISSN: 1535-6108, DOI: 10.1016/J.CCELL.2016.10.018
- BERGERS G. & SONG S.: "The role of pericytes in blood-vessel formation and maintenance", NEURO-ONCOLOGY, vol. 7, 2005, pages 452-464,
- NOMAN M.Z. ET AL.: "Improving cancer immunotherapy by targeting the hypoxic tumor microenvironment: New opportunities and challenges.", CELLS, vol. 8, 1083, 2019,
- F. S. Hodi ET AL: "Bevacizumab plus Ipilimumab in Patients with Metastatic Melanoma", Cancer Immunology Research, vol. 2, no. 7, 21 April 2014 (2014-04-21), pages 632-642, XP055163152, ISSN: 2326-6066, DOI: 10.1158/2326-6066.CIR-14-0053

## Description

### BACKGROUND

Peptides derived from collagen type IV have been described for their potential to inhibit angiogenesis and lymphangiogenesis. Peptide motifs derived from the non-collagenous domain of the α5 fibril of type IV collagen are described in US Patent 9,056,923, WO 2012/079088, and Lee et al., Scientific Reports 4:7139 (2014). For example, a peptide comprising the motif NINNV is described as inhibiting proliferation, migration, and tubule formation of human umbilical vein endothelial cells (HUVEC). Rosca et al., Structure-activity relationship study of collagen derived anti-angiogenic biomimetic peptides. Chem. Biol. Drug Des. 80(1):27-37 (2012).

A better understanding of the biological targets, biological activities, and pharmaceutical properties of these peptides are needed to support and/or direct pharmaceutical uses and product development.

The present disclosure focuses on the therapeutic properties of such peptides in combination with immune checkpoint blockade. Teachings useful to understand the background can be found in Hodi (2014); Cancer Immunol. Res. 2(7):632-642.

### SUMMARY

The invention is defined by the appended claims.

It concerns a pharmaceutical composition comprising a peptide targeting α5β1 and αVβ3 integrins for use in treating cancer. The peptides are derived from the α5 fibril of type IV collagen and have the amino acid sequence according to SEQ ID NO:1-31. The peptidesinhibit signaling through multiple receptors, including vascular endothelial growth factor receptor (VEGFR), hepatocyte growth factor receptor (HGFR), insulin-like growth factor receptor (IGFR), and platelet-derived growth factor receptor (PDGFR).

In particular, the compositions improve immune checkpoint inhibitor therapy by inhibiting angiogenesis and allowing dendritic cell maturation and more robust lymphocyte endothelial trafficking. Preferably, the peptide is formulated with a polymeric nanoparticle or microparticle carrier.

### BRIEF DESCRIPTION OF THE DRAWINGS

**Figure 1** shows that P07 inhibits VEGF, HGF, and IGF signaling in microvascular endothelial cells.
**Figure 2** shows that P07 inhibits retinal detachment caused by excessive vascular leakage in a mouse model.
**Figure 3** shows that P07 inhibits vascular leakage in a VEGF induced leakage model in the rabbit eye. Leakage is compared to untreated, which is set at 1.0.
**Figure 4** shows that P07 inhibits the growth of orthotopic triple negative breast cancer (TBNC) xenografts in a dose dependent manner.
**Figure 5** shows that P07 inhibits neovascularization in orthotopic TNBC xenografts.
**Figure 6** shows the HPLC results for P08 conjugation, demonstrating that P08 was conjugated efficiently to PLGA-PEG-NHS copolymers and quantified via reverse phase HPLC.
**Figure 7** shows that N07 exhibits a Z-average diameter of approximately 70-80 nm. Slight size increase is seen in samples with conjugated P07 on the nanoparticle.
**Figure 8** shows N07 exhibits a negative zeta potential in deionized water, which is very slightly tunable around -25 mV using different end groups on PEG. Neutral is methoxy-terminated PEG. Negative is carboxy-terminated PEG.
**Figure 9** shows the binding of particles to integrin αvβ3 and α5β1.
**Figure 10** shows the binding of particles to integrin αvβ3 with competition from various peptides.
**Figure 11** shows the binding of N07 particles to MDA-MB-231 and MEC cells.
**Figure 12** shows adhesion inhibition assay results measuring the adhesion of cells to plates pre-treated with N07. % refers to amount of PLGA-PEG molecules that have conjugated P07. "+" or "-" refers to the presence or absence of encapsulated P07.
**Figure 13** shows the inhibitory effects of N07 on proliferation of MEC cells. % refers to amount of PLGA-PEG molecules that have conjugated P07. "+" or "-" refers to the presence or absence of encapsulated P07.
**Figure 14** shows adhesion assay results measuring the adhesion of cells to plates pre-treated with P07.
**Figure 15** shows microparticles (MPs) made with 85/15 PLGA and P07, also called M07, using double emulsion technique. Lyophilized samples were imaged with SEM. (A) 0% loading; (B) 0.6% final peptide loading by weight; (C) 1% final loading. Scale shown is 10 µM.
**Figure 16** shows M07, stretched 2.25x. Lyophilized samples were imaged with SEM. (A) blank MPs; (B) M07; (C) blank MPs, zoomed in; (D) M07, zoomed in.
**Figure 17** shows TEM images of peptide loaded NPs nonstretched (left) or 2x stretched (right).
**Figure 18** shows Gel quantification of P07 loading in stretched MPs. Quantified using silver staining and peptide standard. Final P07 w/w ratio ~1%, which is comparable to the pre-stretching P07 w/w ratio of ~1%.
**Figure 19** shows release of P07 from 65/35 PLGA microparticles loaded with P07. Error bars represent standard deviation.

### DETAILED DESCRIPTION

The invention is defined in the claims. The technical disclosure set out below may in some respects go beyond the scope of the claims. Elements of the disclosure which do not fall within the scope of the claims are provided for information.

The present invention in various aspects and embodiments involves pharmaceutical compositions of peptides derived from the α5 fibril of type IV collagen for use in treating cancer in a patient. The peptides have an amino acid sequence chosen from SEQ ID NO:1 - 31. The peptides target α5β1 and αVβ3 integrins, and inhibit signaling through multiple receptors, including vascular endothelial growth factor receptor (VEGFR), hepatocyte growth factor receptor (HGFR), insulin-like growth factor receptor (IGFR), and platelet-derived growth factor receptor (PDGFR).

Peptides targeting integrins include those described in US 9,056,923. According to the present disclosure, a peptide targeting integrins may comprise the amino acid sequence LRRFSTXPXXXXNINNVXNF (SEQ ID NO:1), where X is a standard amino acid or non-genetically encoded amino acid. X at position 7 may be M, A, or G; X at position 9 may be F, A, Y, or G; X at position 10 may be M, A, G, dA, or Nle; X at position 11 may be F, A, Y, G, or 4-ClPhe; X at position 12 and position 18 may be independently selected from Abu, G, S, A, V, T, I, L or Allyl-Gly. The peptide may contain about 30 amino acids or less, or about 25 amino acids of less, or about 24 amino acids, or about 23 amino acids, or about 22 amino acids, or about 21 amino acids, or about 20 amino acids.

Other peptides that target integrins have the amino acid sequence LRRFSTAPFAFIDINDVINF (SEQ ID NO:2), or LRRFSTAPFAFININNVINF (SEQ ID NO:3), or LRRFSTAPFAFIDINDVINW (SEQ ID NO:4), or FTNINNVTN (SEQ ID NO: 5), or FTDINDVTN (SEQ ID NO:6), or an amino acid sequence that is a derivative of any of the foregoing as set forth in SEQ ID NOs: 7-31. The peptide of SEQ ID NO:2 is also referred to herein as P07. The peptide of SEQ ID NO:3 is also referred to herein as P06. The peptide of SEQ ID NO:4 is also referred to herein as P08. The peptide of SEQ ID NO:5 is also referred to herein as P05. The peptide of SEQ ID NO:6 is also referred to herein as P09. The peptide of SEQ ID NO:2 (in comparison to SEQ ID NO:1) has an Aspartic Acid at positions 13 and 16, which improves the physical properties of the peptide without negatively impacting the biological activities. The N- and/or C- termini may optionally be occupied by another chemical group (other than amine or carboxy, e.g., amide or thiol), and which can be useful for conjugation of other moieties, including PEG or PLGA-PEG co-polymers, as described in further detail herein. Peptides may be provided in the form of a pharmaceutically acceptable salt in some embodiments, or complexed with other components or encapsulated in particles for targeted or sustained delivery to particular tissues.

The 20 genetically encoded amino acids can be grouped into the following six standard amino acid groups:
(1) hydrophobic: Met, Ala, Val, Leu, Ile;
(2) neutral hydrophilic: Cys, Ser, Thr; Asn, Gln;
(3) acidic: Asp, Glu;
(4) basic: His, Lys, Arg;
(5) residues that influence chain orientation: Gly, Pro; and
(6) aromatic: Trp, Tyr, Phe.

As used herein, "conservative substitutions" are defined as exchanges of an amino acid by another amino acid listed within the same group of the six standard amino acid groups shown above. For example, the exchange of Asp by Glu retains one negative charge in the so modified polypeptide. In addition, glycine and proline may be substituted for one another based on their ability to disrupt α-helices. Some preferred conservative substitutions within the above six groups are exchanges within the following sub-groups: (i) Ala, Val, Leu and Ile; (ii) Ser and Thr; (ii) Asn and Gln; (iv) Lys and Arg; and (v) Tyr and Phe.

As used herein, "non-conservative substitutions" are defined as exchanges of an amino acid by another amino acid listed in a different group of the six standard amino acid groups (1) to (6) shown above.

The peptide may contain at least one, at least two, or at least three D-amino acids. It may contain from one to about five (e.g., 1, 2, or 3) non-genetically encoded amino acids, which are optionally selected from 2-Aminobutyric acid (Abu), norleucine (Nle), 4-chlorophenylalanine (4-ClPhe), and Allylglycine (AllylGly).

Peptide agents derived from the peptides of SEQ ID NOS: 2 to 6, include:
LRRFSTMPFMF(Abu)NINNV(Abu)NF (SEQ ID NO:7),
LRRFSTMPAMF(Abu)NINNV(Abu)NF (SEQ ID NO:8),
LRRFSTMPFAF(Abu)NINNV(Abu)NF (SEQ ID NO:9),
LRRFSTMPFMA(Abu)NINNV(Abu)NF (SEQ ID NO:10),
LRRFSTMPF(Nle)F(Abu)NINNV(Abu)NF (SEQ ID NO:11),
LRRFSTMPFM(4-ClPhe)(Abu)NINNV(Abu)NF (SEQ ID NO:12),
LRRFSTMPFMFSNINNVSNF (SEQ ID NO:13),
LRRFSTMPFMFANINNVANF (SEQ ID NO:14),
LRRFSTMPFMFININNVINF (SEQ ID NO:15),
LRRFSTMPFMFTNINNVTNF (SEQ ID NO:16),
LRRFSTMPFMF(AllyGly)NINNV(AllyGly)NF (SEQ ID NO: 17),
LRRFSTMPFMFVNINNVVNF (SEQ ID NO:18),
LRRFSTMPFdAFININNVINF (SEQ ID NO:19),
LRRFSTMPFAFININNVINF (SEQ ID NO:20),
LRRFSTAPFAFININNVINF (SEQ ID NO:21),
LRRFSTAPFdAFIDINDVINF (SEQ ID NO:22),
F(Abu)NINNV(Abu)N (SEQ ID NO:23),
FTNINNVTN (SEQ ID NO:24),
FININNVINF (SEQ ID NO:25),
FSNINNVSNF (SEQ ID NO:26),
FANINNVANF (SEQ ID NO:27),
F(AllyGly)NINNV(AllyGly)NF (SEQ ID NO:28),
FVNINNWNF (SEQ ID NO:29),
A(Abu)NINNV(Abu)NF (SEQ ID NO:30), or
(4-ClPhe)(Abu)NINNV(Abu)NF (SEQ ID NO:31).

The peptide may be delivered in the form of nanoparticle and microparticle formulations, either conjugated to the surface or encapsulated. Exemplary particle formulations based on PLGA-PEG polymers are described in detail herein.

The peptide agents can be chemically synthesized and purified using well-known techniques, such as solid-phase synthesis. See US 9,051,349.

The peptides described herein target α5β1 and αVβ3 integrins, and inhibit signaling through multiple receptors, including vascular endothelial growth factor receptor (VEGFR), hepatocyte growth factor receptor (HGFR), insulin-like growth factor receptor (IGFR), and platelet derived growth factor receptor (PDGFR). Integrins are transmembrane receptors that are the bridges for cell-cell and cell-extracellular matrix (ECM) interactions. Signal transduction from integrins effects the chemical composition and mechanical status of the ECM, which controls numerous biological responses such as regulation of the cell cycle, cell shape, and/or motility; or new receptors being added to the cell membrane. This allows rapid and flexible responses to events at the cell surface. There are several types of integrins, and a cell may have several types on its surface. Integrins work alongside other receptors such as cadherins, the immunoglobulin superfamily cell adhesion molecules, selectins and syndecans to mediate cell-cell and cell-matrix interaction. Ligands for integrins include fibronectin, vitronectin, collagen, and laminin.

The invention provides pharmaceutical compositions for use in a method for treating cancer in a patient, by normalizing a tumor microenvironment, and in particular by improving immune checkpoint inhibitor therapy.

The method comprises administering an effective amount of a peptide having the amino acid sequence of any one of SEQ ID NO:1 to 6, or a derivative as set forth in SEQ ID NOs: 7-31 or combination thereof, to a cancer patient undergoing therapy with (or in preparation for therapy) an immune checkpoint inhibitor. Angiogenesis is a drug target for treating cancer. VEGF and its receptor VEGFR2 are important mediators of angiogenesis. Bevacizumab, an antibody that sequesters human VEGF, and other small molecule tyrosine kinase inhibitors that inhibit VEGFR2 have been developed for various types of cancer. In addition to its well-known pro-angiogenic activity, VEGF also functions as an immune suppressor by inhibiting the maturation of dendritic cells. Tumors are thought to produce VEGF both to attract neovasculature and to suppress the immune system by reducing the number of mature immune cells and modulating lymphocyte endothelial trafficking.

Tumors marshal the immune system to promote their own growth. Over the last few years many of the mechanisms by which tumors keep the immune system in check have been deciphered. Many types of tumor cells express surface molecules such as PD-L1 and CTLA-4 that interact with receptors on T-cells that invade the tumor to make them quiescent. These discoveries have allowed the development of so-called "checkpoint inhibitors" such as ipilimumab, tremelimumab, nivolumab, and pembrolizumab as cancer drugs. These drugs are antibodies that interrupt the binding of the tumor cells to the cytotoxic T cells thus freeing them from suppression and allowing them to kill the tumor cells.

At least one study combining bevacizumab and ipilimumab, the antibody that blocks CTLA-4, has been conducted in patients with advanced melanoma (Cancer Immunol Res. 2014 Jul;2(7):632-42). In addition to the effect of blocking VEGF on inflammation; lymphocyte trafficking and immune regulation were also apparent. Based on these studies more clinical trials combining bevacizumab and other anti-angiogenic agents such as small molecule tyrosine kinase inhibitors with checkpoint inhibitors have been initiated.

Other targets for checkpoint inhibitors, for which the peptides and compositions of the present invention may work synergistically, include LAG-3, KIR, OX40L, IDO-1, and TIM-3.

Further, the specificity of the peptide (e.g., SEQ ID NOS 1 to 6, and derivatives as set forth in SEQ ID NOs: 7-31) for α5β1 and αVβ3 integrins, as disclosed herein, suggests other medically important roles for the peptide in cancer therapy. The receptors have been identified as the α5β1 and αVβ3 integrins. Integrins function as co-receptors for many different growth factor receptors. The peptides described herein and derivatives thereof inhibit signaling from the vascular endothelial growth factor receptor (VEGFR2), the hepatocyte growth factor receptor (c-met), and insulin-like growth factor receptor among others. P07, for example, strongly inhibits VEGF induced neovascularization and leakage in mouse models of retinal and choroidal neovascularization and vascular leakage. In addition to promoting angiogenesis, VEGF also causes immunosuppression which is exploited by the tumor to dampen the immune response against it. Since P07 blocks signaling by VEGF, it could effectively act as an immune system booster and thus promote attack on the tumor by the immune system. These data suggest that P07 (as well as other peptides disclosed herein and derivatives) could work well in combination with checkpoint inhibitors. It would help to simultaneously inhibit angiogenesis and strengthen the immune response against tumors by allowing dendritic cell maturation and more robust lymphocyte endothelial trafficking, so that the checkpoint inhibitor could allow infiltrating cytotoxic T-cells to kill tumor cells.

The immune checkpoint inhibitor can be an anti-PD-1 antibody, an anti-PD-L1 antibody, or an anti-CTLA-4 antibody. The methods can be useful against any cancer where immune checkpoint therapy is effective, and especially where the cancer is a sarcoma, carcinoma, or solid tumor cancer selected from germ line tumors, tumors of the central nervous system, breast cancer, prostate cancer, cervical cancer, uterine cancer, lung cancer, ovarian cancer, testicular cancer, thyroid cancer, astrocytoma, glioma, pancreatic cancer, stomach cancer, liver cancer, colon cancer, melanoma (including advanced melanoma), renal cancer, bladder cancer, esophageal cancer, cancer of the larynx, cancer of the parotid, cancer of the biliary tract, rectal cancer, endometrial cancer, squamous cell carcinomas, adenocarcinomas, small cell carcinomas, neuroblastomas, mesotheliomas, adrenocortical carcinomas, epithelial carcinomas, desmoid tumors, desmoplastic small round cell tumors, endocrine tumors, Ewing sarcoma family tumors, germ cell tumors, hepatoblastomas, hepatocellular carcinomas, lymphomas, melanomas, non-rhabdomyosarcome soft tissue sarcomas, osteosarcomas, peripheral primative neuroectodermal tumors, retinoblastomas, rhabdomyosarcomas, and Wilms tumors. In particular, the cancer may be non-small cell lung cancer, melanoma, prostate cancer, metastatic renal cell cancer. Generally, the cancer is positive for PD-1, PD-L1 or CTLA-4, and the checkpoint inhibitor therapy is an agent that inhibits an interaction between PD-1 and PD-L1 or CTLA-4 and B7.

The patient can have either early stage cancer (e.g., stage I or II), or be in later stages (stage III or stage IV). Stage I cancers are localized to one part of the body. Stage II cancers are locally advanced, as are Stage III cancers. Whether a cancer is designated as Stage II or Stage III can depend on the specific type of cancer. For example, stage II can indicate affected lymph nodes on only one side of the diaphragm, whereas stage III indicates affected lymph nodes above and below the diaphragm. The specific criteria for stages II and III therefore differ according to diagnosis. Stage IV cancers have often metastasized, or spread to other organs or throughout the body.

In some cases, the cancer can be non-resectable. A non-resectable cancer is a malignancy which cannot be surgically removed, due either to the number of metastatic foci, or because it is in a surgical danger zone.

In some cases, the patient may be non-responsive or only partially responsive to the immune checkpoint inhibitor alone. The peptide or pharmaceutical composition may be administered with (or during) immune checkpoint inhibitor therapy. Alternatively, the patient may be treated for one to four weeks with the peptide, followed by immune checkpoint inhibitor therapy.

In all such scenarios, the peptide can be administered in a variety of forms depending on the desired route and/or dose.

The peptide can be delivered as a pharmaceutically acceptable salt, and may include any number of carriers known in the art. The term "pharmaceutically acceptable salt" includes salts that are prepared with relatively nontoxic acids or bases. As used herein, "pharmaceutically acceptable carrier" is intended to include, but is not limited to, water, saline, dextrose solutions, human serum albumin, liposomes, hydrogels, microparticles and nanoparticles.

Depending on the specific conditions being treated, the peptide agents may be formulated into liquid or solid dosage forms and administered systemically or locally. The agents may be delivered, for example, in a timed- or sustained-low release form as is known to those skilled in the art. Techniques for formulation and administration may be found in Remington: The Science and Practice of Pharmacy (20th ed.) Lippincott, Williams & Wilkins (2000). Suitable routes may include oral, buccal, by inhalation spray, sublingual, rectal, transdermal, vaginal, transmucosal, nasal or intestinal administration; parenteral delivery, including intramuscular, subcutaneous, intramedullary injections, as well as intrathecal, direct intraventricular, intravenous, intra-articular, intra-sternal, intra-synovial, intra-hepatic, intralesional, intracranial, intraperitoneal, intranasal, or intraocular injections or other modes of delivery.

The form and/or route of administration can vary; the peptide or pharmaceutical composition may be administered parenterally (e.g., by subcutaneous, intravenous, or intramuscular administration), or may be administered directly to the lungs. Local administration to the lungs can be achieved using a variety of formulation strategies including pharmaceutical aerosols, which may be solution aerosols or powder aerosols. Powder formulations typically comprise small particles. Suitable particles can be prepared using any means known in the art, for example, by grinding in an airjet mill, ball mill or vibrator mill, sieving, microprecipitation, spray-drying, lyophilization or controlled crystallization. Typically, particles will be about 10 microns or less in diameter. Powder formulations may optionally contain at least one particulate pharmaceutically acceptable carrier known to those of skill in the art. Examples of suitable pharmaceutical carriers include, but are not limited to, saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. Alternatively, solution aerosols may be prepared using any means known to those of skill in the art, for example, an aerosol vial provided with a valve adapted to deliver a metered dose of the composition. Where the inhalable form of the active ingredient is a nebulizable aqueous, organic or aqueous/organic dispersion, the inhalation device may be a nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer which allows smaller nebulized volumes, e.g. 10 µl to 100 µl.

For injection, the peptides may be formulated and diluted in aqueous solutions, such as in physiologically compatible buffers such as Hank's solution, Ringer's solution, or physiological saline buffer.

The peptides may also be formulated using pharmaceutically acceptable inert carriers into dosages suitable for systemic administration. With proper choice of carrier and suitable manufacturing practice, the compositions of the present disclosure, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the disclosure to be formulated as tablets, pills, capsules, liquids, gels, syrups, slurries, suspensions and the like, for oral ingestion by a subject (e.g., patient) to be treated.

For nasal or inhalation delivery, the peptides may be formulated by methods known to those of skill in the art, and may include, for example, but not limited to, examples of solubilizing, diluting, or dispersing substances such as, saline, preservatives, such as benzyl alcohol, absorption promoters, and fluorocarbons.

The peptides may be formulated with a polymeric nanoparticle or microparticle carrier. For example, the microparticle or nanoparticle may comprise a material having one or more degradable linkages, such as an ester linkage, a disulfide linkage, an amide linkage, an anhydride linkage, and a linkage susceptible to enzymatic degradation. In particular, the microparticle or nanoparticle may comprise a biodegradable polymer or blends of polymers selected from the group consisting of poly(lactic-co-glycolic acid) (PLGA), poly(beta-amino ester) (PBAE), polycaprolactone (PCL), polyglycolic acid (PGA), polylactic acid (PLA), poly(acrylic acid) (PAA), poly-3-hydroxybutyrate (P3HB) and poly(hydroxybutyrate-co-hydroxyvalerate). Nondegradable polymers that are used in the art, such as polystyrene, may be blended with a degradable polymer or polymers from above to create a copolymer system. Accordingly, in some embodiments, a nondegradable polymer is blended with the biodegradable polymer.

Provided herein are nanoparticles comprising PLGA-PEG copolymers and a conjugated peptide targeting integrins. The conjugated peptide is a peptide of any one or more of SEQ ID NO:1 to 31. For example, N07 is a designation used herein for a peptide-conjugated nanoparticle based on P07 that has anti-angiogenic and anti-tumorigenic properties. N07 has anti-angiogenic and anti-tumorigenic activity in vitro, specific binding to the integrin αVβ3 complex, and the ability to carry encapsulated drug cargo.

The nanoparticles may contain an additional drug or targeting agent conjugated to the surface. For example, the nanoparticles may be made from PLGA-PEG-X and PLGA-PEG-Y polymers, where X is said peptide and Y is another drug or targeting agent. The targeting agent may be a tissue selective targeting agent, or may be selective for cancer cells. Such nanoparticles, which have a conjugated peptide and optionally an additional targeting agent, may be used in a treatment of cancer, including solid tumors as described above, and including glioblastoma or breast cancer (including triple negative breast cancer).

The additional targeting agents include antibodies and antigen-binding portions thereof. The various formats for target binding include a single-domain antibody, a recombinant heavy-chain-only antibody (VHH), a single-chain antibody (scFv), a shark heavy-chain-only antibody (VNAR), a microprotein (cysteine knot protein, knottin), a DARPin, a Tetranectin, an Affibody; a Transbody, an Anticalin, an AdNectin, an Affilin, a Microbody, a peptide aptamer, a phylomer, a stradobody, a maxibody, an evibody, a fynomer, an armadillo repeat protein, a Kunitz domain, an avimer, an atrimer, a probody, an immunobody, a triomab, a troybody, a pepbody, a vaccibody, a UniBody, a DuoBody, a Fv, a Fab, a Fab', a F(ab')2, a peptide mimetic molecule, or a synthetic molecule, or as described in US Patent Nos. or Patent Publication Nos. US 7,417,130, US 2004/132094, US 5,831,012, US 2004/023334, US 7,250,297, US 6,818,418, US 2004/209243, US 7,838,629, US 7,186,524, US 6,004,746, US 5,475,096, US 2004/146938, US 2004/157209, US 6,994,982, US 6,794,144, US 2010/239633, US 7,803,907, US 2010/119446, and/or US 7,166,697. See also, Storz MAbs. 2011 May-Jun; 3(3): 310-317.

The nanoparticle can synthesized from poly(lactic-co-glycolic acid) polyethylene glycol (PLGA-PEG) block copolymers of tunable size which are covalently linked to the peptide (e.g., comprising the sequence of any one of SEQ ID NOS:1 to 6, or derivative thereof), or other binding agent as described above. A mix of conjugated and unconjugated polymers in any ratio can be used to create nanoparticles with the desired density of targeting agent on the surface. A description of the tunable characteristics of the particle can be found in Table 1.

For the specific purpose of treating cancer in accordance with the claims, the peptide conjugated to the particle has the amino acid sequence of SEQ ID NO:1, SEQ ID NO:2, SEQ ID NO:3, SEQ ID NO:4, SEQ ID NO:5, or SEQ ID NO:6, or derivative thereof as set forth in SEQ ID NOS:7-31. The nanoparticles can be formed from PLGA-PEG-peptide conjugates, or in in the alternative the peptide is conjugated to pre-formed particles.

As used herein, the term "particle" as used herein is meant to include nanoparticles and microparticles. The term "microparticle" includes particles having at least one dimension in the range of at least about one micrometer (µm). The term "nanoparticle," refers to a particle having at least one dimension in the range of about 1 nm to about 1000 nm, including any integer value between 1 nm and 1000 nm (including about 1, 2, 5, 10, 20, 50, 60, 70, 80, 90, 100, 200, 500, and 1000 nm and all integers and fractional integers in between). Typically the nanoparticle has at least one dimension, e.g. diameter, of about 50 to about 100 nm, and in some cases about 70 to 100 nm.

The particles may be designed to provide desired pharmacodynamic advantages, including circulating properties, biodistribution, and degradation kinetics. Such parameters include size, surface charge, polymer composition, ligand conjugation chemistry, peptide conjugation density, among others. For example, the particles may have a PLGA polymer core, and a hydrophilic shell formed by the PEG portion of PLGA-PEG co-polymers, wherein a portion of the PLGA-PEG polymers have a terminal attachment of the peptide. The hydrophilic shell may further comprise ester-end-capped PLGA-PEG polymers that are inert with respect to functional groups, such as PLGA-PEG-MeOH polymers. In some cases, some or all of the unconjugated polymers may have other terminal groups (such as carboxy) to provide fine tuning of the surface properties.

Peptides described herein can be chemically conjugated to the particles using any available process. Functional groups for peptide conjugation include PEG-COOH, PEG-NH₂, PEG-SH. See, e.g., Hermanson, BlOCONJUGATE TECHNIQUES, Academic Press, New York, 1996. Activating functional groups include alkyl and acyl halides, amines, sulfhydryls, aldehydes, unsaturated bonds, hydrazides, isocyanates, isothiocyanates, ketones, and other groups known to activate for chemical bonding. Alternatively, peptides can be conjugated through the use of a small molecule-coupling reagent. Non-limiting examples of coupling reagents include carbodiimides, maleimides, N-hydroxysuccinimide esters, bischloroethylamines, bifunctional aldehydes such as glutaraldehyde, anhydrides and the like.

The nanoparticles may have a core (PLGA) that can be tuned for a specific biodegradation rate in vivo (by adjusting the LA:GA ratio and/or molecular weight of the PLGA polymer). In some cases, the PLGA is based on a LA:GA ratio of from 20:1 to 1:20, including compositions of L/G of: 5/95, 10/90, 15/85, 20/80, 25/75, 30/70, 35/65, 40/60, 45/55, 50/50, 55/45, 60/40, 65/35, 70/30, 75/25, 80/20, 85/15, 90/10, or 95/5. PLGA degrades by hydrolysis of its ester linkages. The time required for degradation of PLGA is related to the ratio of monomers: the higher the content of glycolide units, the lower the time required for degradation as compared to predominantly lactide units. In addition, polymers that are end-capped with esters (as opposed to the free carboxylic acid) have longer degradation half-lives.

Typically, the PLGA polymers for fabricating nanoparticles have a molecular weight in the range of about 10K to about 70K, such as about 20K, about 25K, about 30K, about 40K, about 50K, about 60K, or about 70K, to provide tunable particle size. The PLGA polymers used for fabricating microparticles have a molecular weight in the range of about 20K to about 200K, such as from 100K to about 200K. The PEG portion of the polymer is generally in the range of 2K to 5K. The ratio of PLGA-PEG-peptide and unconjugated PLGA-PEG typically ranges from about 1:20 to about 20:1, such as from about 1:15 to about 15:1, or about 1:10 to about 10:1, or about 1:5 to about 5:1, or about 1:2 to about 2:1. In some cases, the ratio of PLGA-PEG-peptide and unconjugated copolymers is about 1:1. In some cases, at least 50% of the polymers have conjugated peptide. Thee nanoparticle can have a size (average diameter) within the range of about 50 to about 200 nm, or within the range of about 50 to about 100 nm. The nanoparticle can have a zeta potential in deionized water within the range of about -5 mV to about -40 mV, and in some cases, from about -10 mV to about -30 mV (e.g., about -20, about -25, or about -30 mV).

While the nanoparticle is typically substantially spherical, it may also be non-spherical.

There are various physical and chemical properties that can affect how a material interacts with a biological system. In the case of microparticle and nanoparticle based materials, the choice of material, the size distribution, and the shape distribution of the particles are all critical parameters affecting the particles' activity. It has been previously shown that both the size and shape of a particle can affect the way the particle interacts with various cells of the body. For example, the shape of the particle can affect how well various cell types can uptake the particle, where an ellipsoidal particle is usually more difficult for a cell to uptake than a spherical particle. Stretching the shape of the particles can therefore reduce unwanted uptake of particles, such as by the immune system cells, thereby extending the half-life of the particles in the body. The size of the particle also affects the ability of cells to uptake and interact with the particles. Optimization of the activity of a particle based system can therefore be achieved by tuning the size and shape distribution of the particles.

Preferably, the dimensions of the nanoparticle and/or process for stretching the particles in as disclosed in WO 2013/086500.

In particular cases, the three-dimensional microparticle or nanoparticle comprises a prolate ellipsoid, wherein the dimension (a) along the x-axis is greater than the dimension (b) along the y-axis, and wherein the dimension (b) along the y-axis is substantially equal to the dimension (c) along the z-axis, such that the prolate ellipsoid can be described by the equation a > b = c. In other cases, the ellipsoid is a tri-axial ellipsoid, wherein the dimension (a) along the x-axis is greater than the dimension (b) along the y-axis, and wherein the dimension (b) along the y-axis is greater than the dimension (c) along the z-axis, such that the tri-axial ellipsoid can be described by the equation a > b > c. In yet other cases, the ellipsoid is an oblate ellipsoid, wherein the dimension (a) along the x-axis is equal to the dimension (b) along the y-axis, and wherein the dimension (b) along the y-axis is greater than the dimension (c) along the z-axis, such that the oblate ellipsoid can be described by the equation a = b > c. Asymmetrical particles do not include cases in which a = b = c.

The microparticle or nanoparticle can an aspect ratio ranging from about 1.1 to about 5. Alternatively, the aspect ratio has a range from about 5 to about 10. In some cases, the aspect ratio has a range from about 1.5 to about 3.5.

In some some cases, the particle is a microparticle that encapsulates the integrin-targeting peptides disclosed herein. The particle may or may not contain peptide conjugated to its surface. In these cases, the particle can provide a long acting drug depot, to provide a sustained release of the peptides. Exemplary particle formats include those described in WO 2014/197892. In some cases, particles do not incorporate poly(beta-amino ester) (PBAE), and thus the polymers consist essentially of PLGA-PEG block co-polymers. In these or other cases, the particle may have a size (average diameter) in the range of 1 µm to 500 of µm, such as in the range of about 1 µm to about 250 µm. The particles can be injected from about once daily to about once every six months, or about weekly or about monthly, depending on the duration of the sustained peptide or drug release.

### EXAMPLES

### Example 1: P07 Inhibits Signaling of VEGF, HGF and IGF

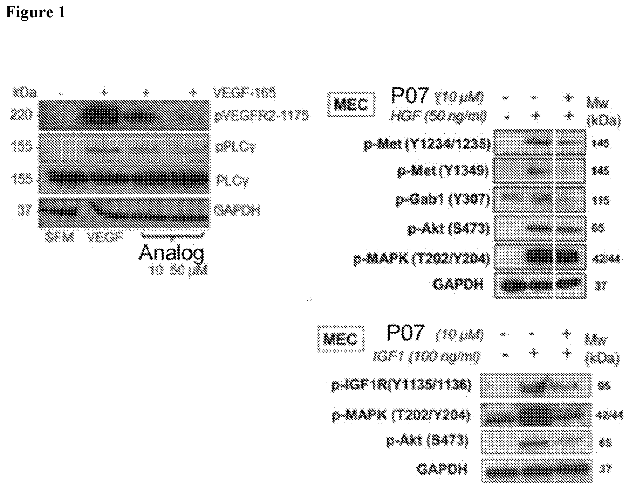

The targets of P07 were identified to be the α5β1 and αVβ3 integrins. Integrins function as co-receptors for many growth factor receptors such as VEGFR, hepatocyte growth factor receptor (HGFR or c-met), insulin-like growth factor receptor (IGFR), and platelet derived growth factor receptor (PDGFR). Consistent with this mechanism, P07 was found to inhibit signaling from these receptors (**Fig. 1**).

These receptors and others are involved in angiogenesis and microvascular permeability. This multi-factorial inhibition makes it likely that diseases involving multiple mechanisms can be effectively treated with P07 and its derivatives.

### Example 2: P07 Inhibits Neovascularization in Multiple Ocular Models

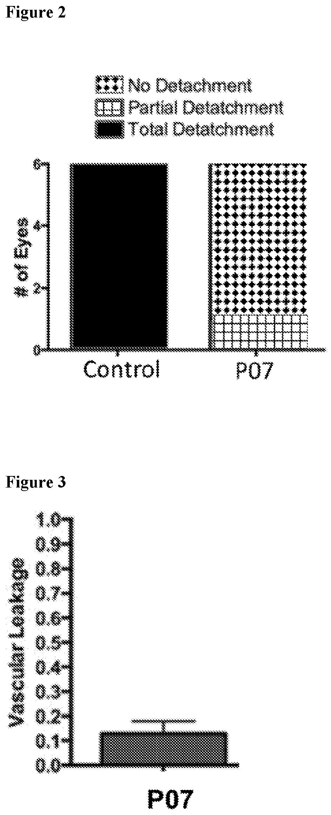

P07 was found to inhibit vascular leakage in a transgenic mouse over-expressing the human form of vascular endothelial growth factor (VEGF) in the retina. The vascular leakage in this model is so severe that the blood that pools behind the retina causes retinal detachment. In this model, P07 almost completely blocked retinal detachment (**Fig. 2**).

P07 was also tested in a model of edema in the rabbit eye. In this model human VEGF was injected directly into the eye causing the local vasculature to be leaky. The extent of this leak was assessed by measuring the amount of fluorescence in the eye resulting from the leakage of sodium fluorescein administered intravenously 3 days after VEGF injection. When P07 was present at about 50 µg in the eye prior to VEGF injection, the vascular leakage was dramatically inhibited (**Fig. 3**). These results suggest that P07 is a potent anti-edemic agent *in vivo.*

### Example 3: P07 Inhibits Cancer Tissue Growth

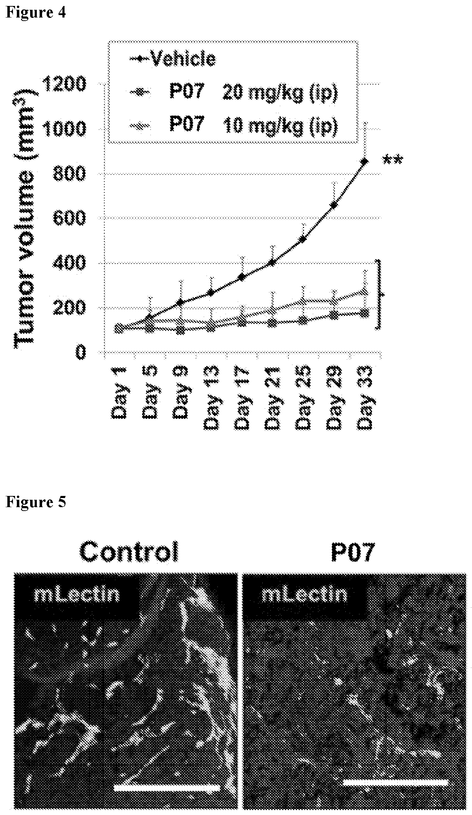

P07 inhibits the growth of orthotopic triple-negative breast cancer (TNBC) xenografts (**Fig. 4**) and small cell lung cancer (SCLC) and glioblastoma xenografts (not shown). The responding tumors have dramatically diminished vasculature (**Fig. 5**).

These results indicate that P07 may have synergistic effects when combined with immune checkpoint inhibitors. P07 and an immune checkpoint inhibitor can simultaneously inhibit angiogenesis and strengthen the immune response against tumors by allowing dendritic cell maturation and more robust lymphocyte endothelial trafficking and the checkpoint inhibitor would allow infiltrating cytotoxic T-cells to kill tumor cells.

### Example 4: The Properties of P07-Conjugated Nanoparticle (N07)

N07 is a peptide-conjugated nanoparticle that has anti-angiogenic and anti-tumorigenic properties. N07 specifically binds to the integrin αVβ3 complex, and has the ability to carry encapsulated drug cargo.

N07 was synthesized from poly(lactic-co-glycolic acid) polyethylene glycol (PLGA-PEG) block copolymers of tunable size which are covalently conjugated with N07. A mix of conjugated and unconjugated polymers in any ratio can be used to create nanoparticles with the desired density of P07 on the surface. A description of the tunable characteristics of the particle is shown in Table 1.

**Table 1**

| **Tunable Parameter** | **Applicable Range** |
|---|---|
| Molecular weight of PLGA | 10 kDa - 70 kDa |
| Molecular weight of PEG | 2 kDa - 5 kDa |
| Percent of polymers conjugated to peptide | 0 - 100% |

N07 was synthesized from the P07 peptide and PLGA-PEG block copolymers. P07 was produced by solid state synthesis at New England Peptide and its purity assessed by HPLC/MS. The peptide has an amine at the N-terminus and an amide at the C-terminus. PLGA-PEG block copolymers were purchased from PolySciTech^{®} and purity and molecular weight was assessed by gel permeation chromatography (GPC) and Fourier transform infrared spectroscopy (FTIR).

For conjugation, P07 was dissolved at 100 mg/ml in DMSO and added to 170 mg/ml NHS-functionalized PLGA-PEG (PLGA-PEG-NHS) in DMF. 40 molar excess of diisopropylethylamine (DTPEA) was added to the mixture and stirred overnight at room temperature. The mixture was then added dropwise to a cold mixture of ether and methanol and spun down at 22,000 x g. The pellet was then repeatedly washed with methanol and spun down to remove unreacted peptide. The supernatant was discarded, and the pellet was left to dry under vacuum for several hours to yield solid PLGA-PEG-SP2043. Other cargo, such as drugs and dyes, can also be conjugated to PLGA-PEG via a similar process.

A mixture of PLGA-PEG-P07 and PLGA-PEG was dissolved in DMF at 10 mg/ml and added dropwise to deionized water under magnetic stirring to form nanoparticles in a process called nanoprecipitation. Desired cargo was added to the DMF mixture prior to nanoprecipitation, which resulted in a nanoparticle with loaded cargo in the hydrophobic PLGA core. After several hours of stirring, the particles were filtered and concentrated with Ultra Centrifugation Columns (EMD Millipore, UFC810096).

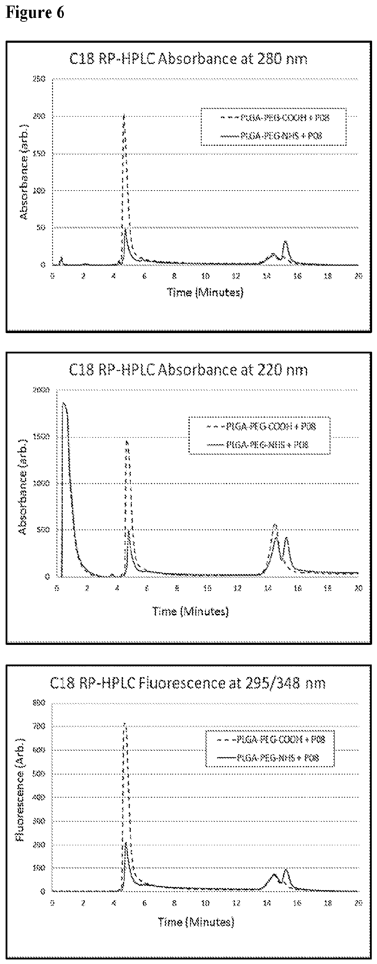

HPLC was used to assess the efficiency of the conjugation of PLGA-PEG-NHS to P07. In some cases, the c-terminal F was replaced with W to allow reading of absorbance and fluorescence from the W residue. PLGA-PEG-P07 from the reaction mixture prior to precipitation in ether and methanol was diluted in DMSO and run through an Agilent Poroshell 300 column in a water-acetonitrile mobile phase. Peptide was detected via absorbance at 220 nm (peptide backbone) and 280 nm (tryptophan, W), and via fluorescence at 295/348 nm excitation/emission (tryptophan). The amount of unreacted P07 was determined via integration of the free P07 peak. The PLGA-PEG-P07 reaction mixture was compared to a control reaction mixture of PLGA-PEG-COOH or PLGA-mPEG and P07, neither of which will undergo a reaction to form PLGA-PEG-P07. Reacted P07 would not contribute to the free P07 peak, so a reduction in the P07 peak relative to the control reaction indicates conjugation to the PLGA-PEG-NHS copolymer. Results were compared to a standard curve of free peptide to ensure all integration values fell within a linear range of peptide concentration. The results are shown in Table 2 and **Fig. 6****.**

**Table 2**

| Signal for Quantification | Conjugation Efficiency |
|---|---|
| Fluorescence (295/348 nm) | 0.869 |
| Absorbance (280 nm) | 0.920 |
| Absorbance (220 nm) | 0.854 |

LavaPep^{™} Characterization: The LavaPep^{™} peptide quantification kit (Gel Company, LP022010) was used to directly detect peptide on the surface of N07. Particles at 3-5 mg/ml in ultrapure water were incubated for 1 hour in the dark with the LavaPep working solution in 96-well plates. The epicocconone dye in the LavaPep working solution interacted with the Arg residues on the peptide to become highly fluorescent. Fluorescence was read at 530/590 nm on a Biotek HT Synergy plate reader. The signal from the nanoparticles was compared to a standard dilution curve of known amounts of free peptide. The quantification results are shown in Table 3, demonstrating that SP2043 was conjugated efficiently to PLGA-PEG-NHS copolymers and incorporated into nanoparticles.

**Table 3**

| Surface percentage SP2043 | Average Signal | Corresponding Peptide (µg) | Peptide Standard Deviation (µg) | Conjugation Efficiency |
|---|---|---|---|---|
| 100 | 1992 | 14.80 | 0.538 | 0.889 |
| 50 | 1432 | 7.01 | 0.249 | 0.842 |
| 0 | 0 | 0 | - | - |

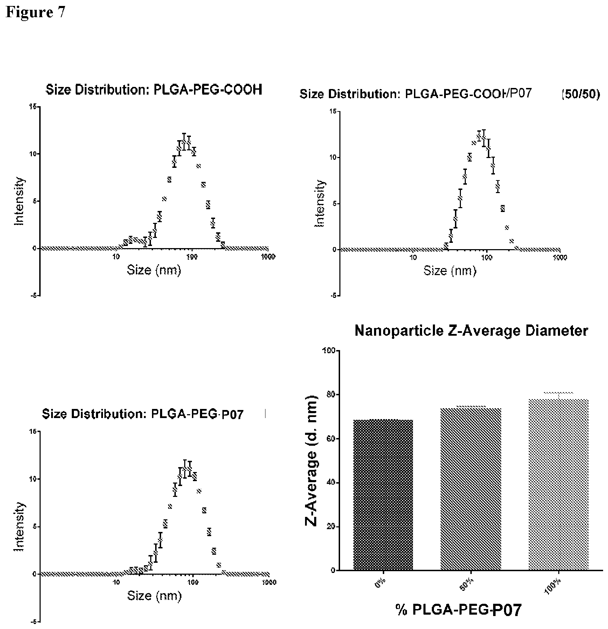

*Size Characterization*: N07 was suspended in water at 1 mg/ml and analyzed using a Malvern Zetasizer Nano ZS90. Z-average diameter and intensity-based size distribution were measured and recorded. The results are shown in Table 4 and **Fig. 7****,** demonstrating that N07 exhibits a Z-average diameter of approximately 70-80 nm and slight size increase was seen in samples with conjugated P07 on the nanoparticle.

**Table 4**

| Sample | Z-Average (d. nm) | PDI |
|---|---|---|
| PLGA-PEG-COOH | 68.24 ± 0.26 | 0.23 |
| PLGA-PEG-COOH/P07 (50/50) | 73.39 ± 1.03 | 0.18 |
| PLGA-PEG-P07 | 77.59 ± 2.65 | 0.29 |

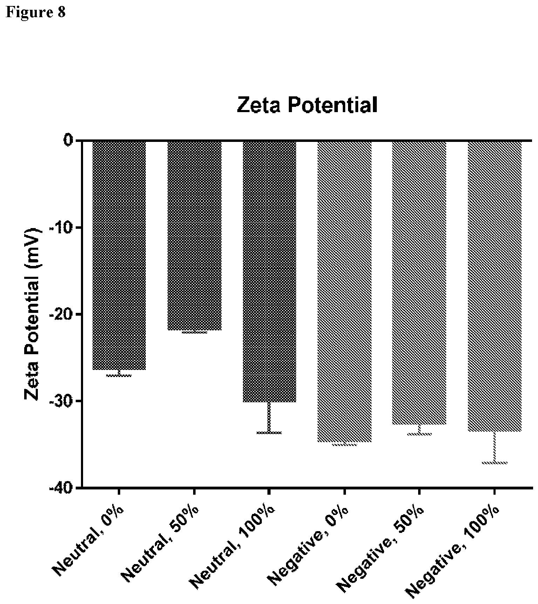

*Zeta-Potential*: N07 was suspended in ultrapure water at 1 mg/ml and analyzed using a Malvern Zetasizer Nano ZS90. Surface zeta potential was measured and recorded. The results are shown in **Fig. 8****,** demonstrating that N07 exhibits a negative zeta potential in deionized water, which is very slightly tunable around 25 mV using different end groups on PEG. In **Fig. 8****,** neutral is methoxy-terminated PEG and negative is carboxy-terminated PEG.

### Example 5: Binding ofPLGA-PEG-P07 to Integrin αvβ3 and Integrin α5β1 Targets

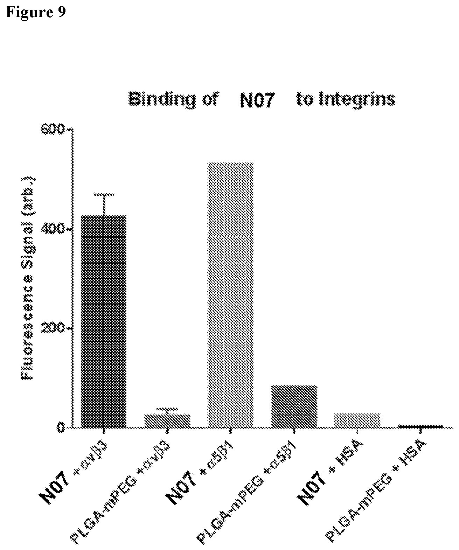

Particles were prepared as described above, made either completely of PLGA-PEG-P07 or PLGA-mPEG. Integrin αvβ3, integrin α5β1, and human serum albumin were labeled with an Alexafluor 488 TFP-ester according to the manufacturer's instructions. Particles were incubated at room temperature with the integrin in PBS overnight alongside a control sample with integrins or HSA in PBS without particles. Particles were then separated from free integrins or HSA protein via SEC centrifugal spin columns using Sephacryl S-500 HR media. After separation, fluorescence signal was measured on a Biotek Synergy HT microplate reader to assess the amount of integrin brought through the SEC media by the particles. The results are shown in **Fig. 9****.**

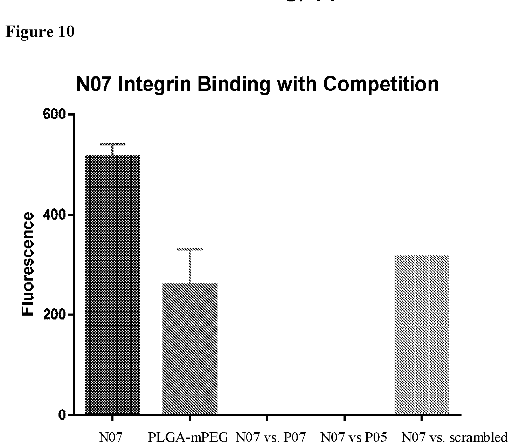

The above protocol was repeated for binding to integrin αvβ3, but with the addition of competition samples. In brief, various peptides (P07 and a partial scramble of the sequence of P07 that has been shown to be inactive) were added to the solution of targeted particles and Alexafluor 488-labeled free integrins at 100 times excess and incubated at room temperature overnight. The solutions were separated via SEC centrifugal spin columns using Sephacryl S-500 HR media. As before, fluorescence was used to assess the amount of integrin brought through the SEC media by the particles. The results are shown in **Fig. 10****.**

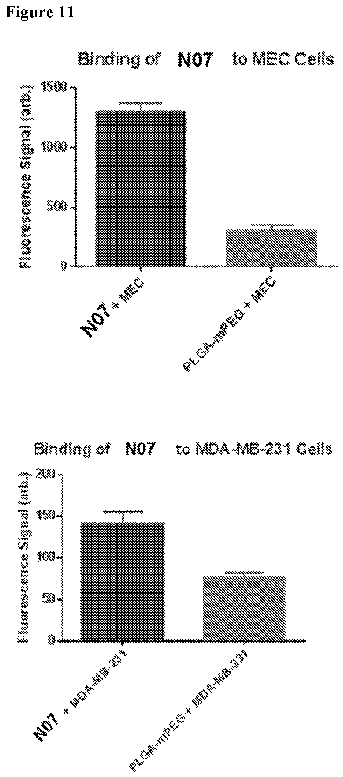

Nanoparticles were tested for binding to MDA-MB-231 and microvascular endothelial cells (MEC) Cells. Particles were prepared as above with the addition of 1% TAMRA dye by weight. After nanoprecipitation, the particles were concentrated with Amicon ultracentrifugation filters (MWCO 100,000) and filtered with SEC centrifugal spin columns using Sephacryl S-500 HR media to remove free TAMRA dye or other free polymer or peptide materials. Cells were then incubated with nanoparticles at 100,000 cells/ml and 1 mg/ml particle. Nanoparticles were made from either targeted PLGA-PEG-P07 polymers or untargeted PLGA-mPEG polymers. After 1 hour incubation at 37 °C, the cells were spun down in the centrifuge and the supernatant was removed. Cells were resuspended in PBS and the resulting signal was on a Biotek Synergy HT microplate reader to assess the amount of fluorescent particle brought down with the cells. The results are shown in **Fig. 11****.**

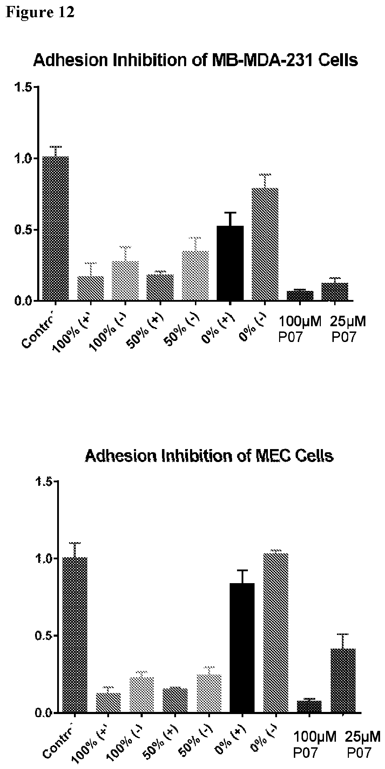

Particles were tested for their ability to inhibit adhesion of MB-MDA-231 cells and microvascular endothelial cells (MEC). Prior to use in *in vitro* assays, particles were transferred from ultrapure water to appropriate media using ultracentrifugation columns, concentrated to 10 mg/ml in media, and added to 96-well plates. Media alone and media with 100 µM and 25 µM AXT201 (a known inhibitor of adhesion) were added to plates as positive and negative controls. MDA-MB-231 or MEC cells were added at 20,000 cells/well to the particles, peptides, and media. The 96-well plate was incubated for approximately 2 hours at 37 °C and 5% CO₂. Wells were then washed twice with DPBS with Ca²⁺ and Mg²⁺ and then filled with media containing 4 µg/ml Calcein AM dye. Plates were then incubated for 30 minutes and washed again with DPBS with Ca²⁺ and Mg²⁺ . Fluorescence was then read on a Biotek Synergy HT at 485/528 nm excitation/emission to quantify the number of cells adhered to the surface of the well. The results are shown in **Fig. 12****,** demonstrating that N07 has anti-adhesion activity against MDA-MD-231 tumor cells and MEC cells. In Fig. 12, the % refers to amount of PLGA-PEG molecules that have conjugated P07. "+" or "-" refers to the presence or absence of encapsulated P07.

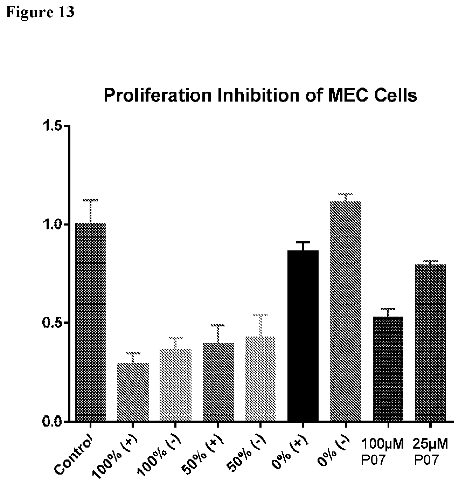

Particles were tested for inhibition of MEC proliferation. Colorimetric based proliferation assay using the MTT Vybrant Assay Kit were carried out on MEC cells. 2000 cells/well were plated in 96-well plates in phenol red-free ECM-2MV media and allowed to adhere over 18-20 hours. Original media with no particles was replaced with N07 particles suspended in media at 5 mg/ml or AXT201 peptide in media or media alone. After four days, media was replaced with 100 ul MTT reagent as per the manufacturer's recommendations. After four hours, 100 ul of SDS solution was added to each well and incubated at 37 °C for another four hours. Absorbance was read at 570 nm on a Biotek Synergy HT plate reader to capture the change from MTT to formazan by mitochondrial reductase in the living cells. The results are shown in **Fig. 13****,** demonstrating that N07 has anti-proliferation activity against MEC cells. In Fig. 13, the % refers to amount of PLGA-PEG molecules that have conjugated P07. "+" or "-" refers to the presence or absence of encapsulated P07.

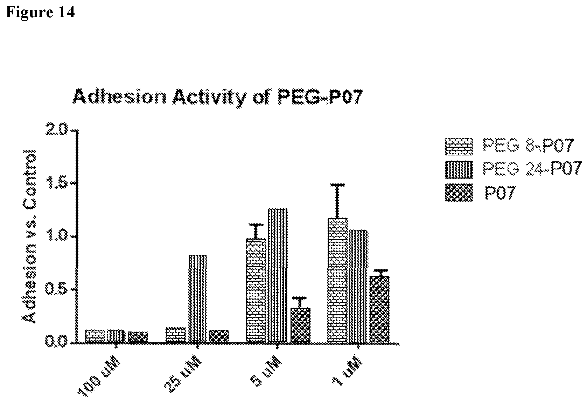

The ability of PEG-P07 conjugates to inhibit adhesion was tested. NHS-functionalized PEG 8 and PEG 24 were dissolved in DMF at approximately 150 mg/ml. Peptide was added at a 1:1 molar ratio in DMSO at 100 mg/ml along with a 40 fold molar excess of DIPEA. The mixture was precipitated in cold ether and methanol and washed several times to remove DIPEA, solvents, and free PEG. The resulting mixture was then used in an adhesion assay using MDA-MB-231 cells as described above alongside positive and negative controls. The results are shown in **Fig. 14****,** demonstrating the adhesion activity of PEG-P07.

### Example 6: Stretching the Peptide-Conjugated Particles

The size and shape of particles were manipulated for the optimization of the activity of the particles. The particles were made and stretched successfully. The peptides remain stable throughout the process, with peptide loading remaining the same before and after the stretching protocol.

*Microparticle formation*: Poly(lactide-co-glycolide), PLGA, was first dissolved into dichloromethane, DCM, at 20 mg/mL in a test tube and vortexed to fully dissolve. Peptide stock of P07 in dimethylsulfoxide, DMSO (20 mg/mL) was micropipetted to the PLGA/DCM solution. The initial mass ratio of peptide to PLGA can vary; such as 1:50 and 1:20 peptide:PLGA. For blank microparticle, equivalent volume of DMSO only was pipetted. The mixture was sonicated with the test tube on ice. Sonication was performed with an amplitude setting of '30', which equals approximately 5-10 W, for 20 seconds. This primary emulsion was immediately poured into 50 mL of 1% poly(vinyl alcohol), PVA, solution and homogenized at 3.6-3.8 krpm for 1 minute. The full volume was then transferred to 100 mL of 0.5% PVA solution and stirred in a chemical hood for about 3.5 hours. Three wash steps were then performed. For each wash step, the microparticle solution was centrifuged at 4°C, 4 krpm, for 5 minutes, and then the supernatant was removed. Subsequently, 40 mL of refrigerated Milli-Q water was added, the microparticle pellet was resuspended and the washing steps were repeated. After the last centrifugation step, 5 mL of water was added to resuspend the sample. Samples were snap frozen in liquid nitrogen and immediately placed in a lyophilizer. Following lyophilization, all microparticles were stored at -20 °C.

*Nanoparticle formation*: PLGA was first dissolved into DCM, at desired concentration (usually 20 mg/mL or 40 mg/mL), in a test tube and vortexed to fully dissolve. Peptide stock, such as P07, in DMSO (20 mg/mL) was micropipetted to the PLGA/DCM solution. The mass ratio of peptide to PLGA can vary. An exemplary formulation is 1:50 peptide:PLGA. For blank nanoparticle, an equivalent volume of DMSO only was pipetted. The mixture was sonicated with the test tube on ice. Sonication (Misonix) was performed with an amplitude setting of '30', which equals approximately 5-10 W, for 20 seconds. This primary emulsion was immediately poured into 50 mL of 1% PVA solution and sonicated at an amplitude setting of anywhere from '30' to '100' for 2 minutes on ice. The full volume was then transferred to 100 mL of 0.5% PVA solution and stirred in a chemical hood for ~3.5 hours. Three wash steps were then performed. For each wash step, the nanoparticle solution was centrifuged at 4°C, 17 krpm, for 10 minutes, and then the supernatant was removed. Subsequently, 30 mL of refrigerated Milli-Q water was added, the nanoparticle pellet was resuspended and the washing steps were repeated. After the last centrifugation step, 5 mL of water was added to resuspend the sample. Samples were snap frozen in liquid nitrogen and immediately placed in a lyophilizer. Following lyophilization, all nanoparticles were stored at -20 °C.

*Microparticle Stretching*: Lyophilized PLGA particles were dissolved in a 10% PVA/ 2% glycerol solution at a concentration of 2.5 mg/mL and 10 mL of this solution was deposited into rectangular petri dishes to dry overnight. The resulting film was cut to size and loaded in between two aluminum mounts and heated up to 90 °C. The film length was measured and the film was stretched slowly to produce the desired fold of stretch (e.g. 2 fold stretched ellipsoidal particles) using custom made stretching device. The film was then allowed to cool down to room temperature and was removed from the aluminum blocks. The PVA film was dissolved in water and the resulting particle suspension was washed 3x. The particles were lyophilized prior to use.

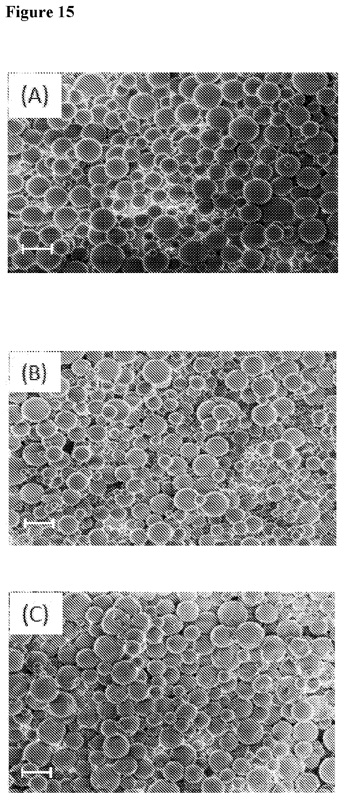

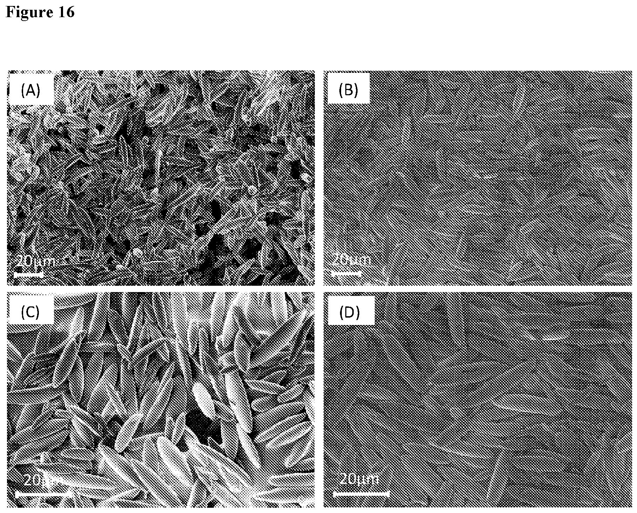

*SEM and TEM characterization*: For scanning electron microscope (SEM), Lyophilized particles were placed on carbon tape (Electron Microscopy Sciences, Hatfield, PA) placed on aluminum mounts. Samples were sputtered with gold-palladium, and SEM imaging was performed with a LEO/Zeiss FESEM at the JHU School of Medicine MicFac. Sizing of microparticle samples was performed with ImageJ analysis of SEM images. The results are shown in **Fig. 15****.** Stretched particles are shown in **Fig. 16****.**

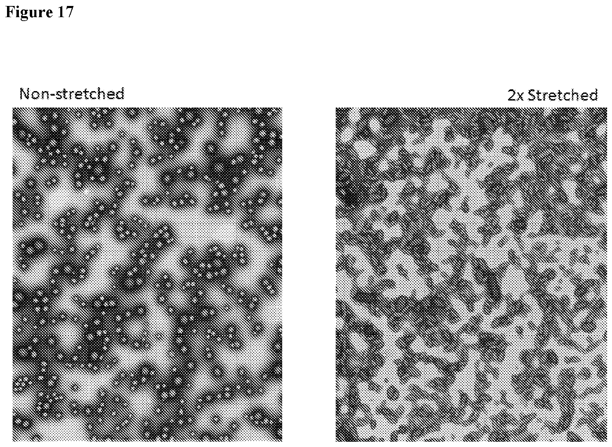

For transmission electron microscopy (TEM), nanoparticles were first resuspended in water at 1mg/mL. 10 uL of sample was dropped onto carbon coated copper grids and left to dry in chemical hood for 2 hours. Unstained TEM imaging was then performed using the Philips CM120 system. The results are shown in **Fig. 17****,** which shows peptide loaded NPs nonstretched (left) or 2x stretched (right).

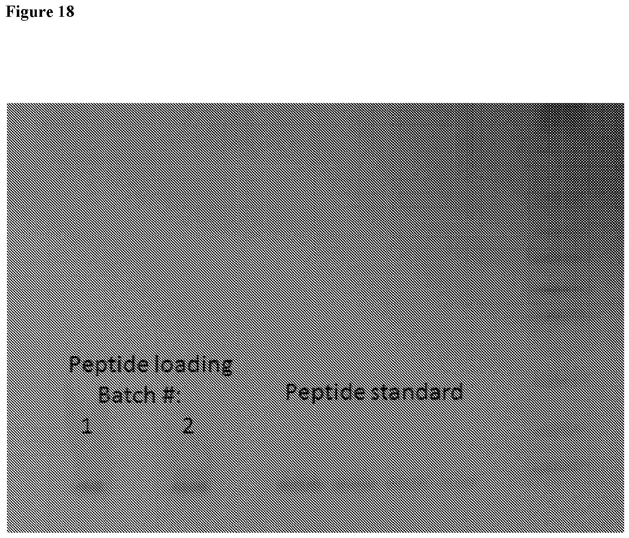

*Microparticle loading and release quantification*: To measure loading, a known mass of microparticles was dissolved in DMSO. For peptide loaded microparticles, and corresponding blank microparticles, quantification was performed by running gel electrophoresis (Bio-Rad Mini-PROTEAN system) and silver stain analysis. A 12-well 10-20% Mini-PROTEAN tris-tricine gel was used, along with 10x tris/tricine/SDS running buffer diluted to 1x in Milli-Q water. Each gel contained a standard series of a known amount of peptide. The peptide standard series included 0, 62.5, 125, 250, and 500 ng of peptide per well. The remaining wells included a protein standard, and the microparticle samples, both peptide loaded and blanks as controls. The DMSO samples were mixed 1:1 by volume with sample buffer. Sample buffer was made of 24% glycerol in 1x PBS. Gel electrophoresis was run until the 2.5 kDa band of the protein standard traveled approximately two-thirds of the way down the gel. The silver stain protocol was followed for gel staining. For the development step, the stop solution was added once the lowest peptide standard (in this case 62.5 ng) began to appear. Gel images were captured with a digital camera and analyzed with ImageJ using gel band intensity quantification functionality. The results are shown in **Fig. 18****.**

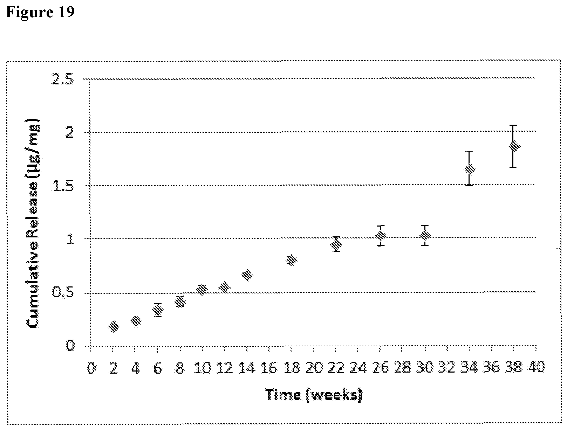

To measure release, a known mass of microparticles was suspended in 1x PBS, placed on a shaker in a 37 °C oven. At various time points, samples were centrifuged for 5 min at ~2.5 krcf. Supernatant was collected and stored at -80 °C, and fresh PBS was added to samples. Quantification of peptide released into the supernatant was performed by running gel electrophoresis, silver staining, and gel band analysis. The results are shown in **Fig. 19****.**

## Claims

1. A pharmaceutical composition comprising a peptide targeting α5β1 and αvβ3 integrins for use in treating cancer in a patient, wherein:
(a) the peptide has the amino acid sequence of SEQ ID NO:1 to 6, or is a derivative thereof that is a peptide of SEQ ID NOs: 7 to 31; and
(b) an effective amount of said peptide is to be administered to a cancer patient for one to four weeks, followed by an immune checkpoint inhibitor therapy.

2. The composition for the use of claim 1, wherein the peptide is administered at least once daily from one to four weeks.

3. The composition for the use of claim 1, wherein the checkpoint inhibitor therapy is an agent that inhibits an interaction between PD-1 and PD-L1; or between CTLA-4 and B7.

4. The composition for the use of claim 1, wherein the immune checkpoint inhibitor targets LAG-3, KIR, OX40L, IDO-1, and TIM-3.

5. The composition for the use of claim 1, wherein the patient is non-responsive or only partially responsive to the immune checkpoint inhibitor alone.

6. The composition for the use of claim 1, wherein the patient has an early stage cancer, or a late stage cancer, or wherein the cancer is non-resectable.

7. The composition for the use of claim 1, wherein the cancer is a sarcoma, carcinoma, or solid tumor cancer selected from germ line tumors, tumors of the central nervous system, breast cancer, prostate cancer, cervical cancer, uterine cancer, lung cancer, ovarian cancer, testicular cancer, thyroid cancer, astrocytoma, glioma, pancreatic cancer, stomach cancer, liver cancer, colon cancer, melanoma, renal cancer, bladder cancer, esophageal cancer, cancer of the larynx, cancer of the parotid, cancer of the biliary tract, rectal cancer, endometrial cancer, squamous cell carcinomas, adenocarcinomas, small cell carcinomas, neuroblastomas, mesotheliomas, adrenocortical carcinomas, epithelial carcinomas, desmoid tumors, desmoplastic small round cell tumors, endocrine tumors, Ewing sarcoma family tumors, germ cell tumors, hepatoblastomas, hepatocellular carcinomas, lymphomas, melanomas, non-rhabdomyosarcoma soft tissue sarcomas, osteosarcomas, peripheral primitive neuroectodermal tumors, retinoblastomas, rhabdomyosarcomas, and Wilms tumors.

## Patentansprüche

1. Eine pharmazeutische Zusammensetzung, umfassend ein Peptid, das auf α5β1- und αvβ3-Integrine abzielt, zur Verwendung bei der Behandlung von Krebs bei einem Patienten, wobei:
(a) das Peptid die Aminosäuresequenz von SEQ ID NO:1 bis 6 aufweist oder ein Derivat davon ist, das ein Peptid der SEQ ID NOs: 7 bis 31 ist; und
(b) eine wirksame Menge des Peptids einem Krebspatienten eine bis vier Wochen lang verabreicht werden soll, gefolgt von einer Immun-Checkpoint-Inhibitor-Therapie.

2. Die Zusammensetzung für die Verwendung nach Anspruch 1, wobei das Peptid mindestens einmal täglich über einen Zeitraum von einer bis vier Wochen verabreicht wird.

3. Die Zusammensetzung zur Verwendung nach Anspruch 1, wobei die Checkpoint-Inhibitor-Therapie ein Agens ist, das eine Wechselwirkung zwischen PD-1 und PD-L1 oder zwischen CTLA-4 und B7 inhibiert.

4. Die Zusammensetzung zur Verwendung nach Anspruch 1, wobei der Immun-Checkpoint-Inhibitor auf LAG-3, KIR, OX40L, IDO-1 und TIM-3 abzielt.

5. Die Zusammensetzung zur Verwendung nach Anspruch 1, wobei der Patient auf den Immun-Checkpoint-Inhibitor allein nicht oder nur teilweise anspricht.

6. Die Zusammensetzung zur Verwendung nach Anspruch 1, wobei der Patient Krebs im Frühstadium oder im Spätstadium hat oder wobei der Krebs nicht resezierbar ist.

7. Die Zusammensetzung zur Verwendung nach Anspruch 1, wobei es sich bei dem Krebs um ein Sarkom, ein Karzinom oder einen soliden Tumorkrebs handelt, der ausgewählt ist aus Keimbahntumoren, Tumoren des Zentralnervensystems, Brustkrebs, Prostatakrebs, Gebärmutterhalskrebs, Gebärmutterkrebs, Lungenkrebs, Eierstockkrebs, Hodenkrebs, Schilddrüsenkrebs, Astrozytom, Gliom, Bauchspeicheldrüsenkrebs, Magenkrebs, Leberkrebs, Dickdarmkrebs, Melanom, Nierenkrebs, Blasenkrebs, Speiseröhrenkrebs, Kehlkopfkrebs, Ohrspeicheldrüsenkrebs, Krebs des Gallenganges, Rektumkarzinom, Endometriumkarzinom, Plattenepithelkarzinom, Adenokarzinom, kleinzellige Karzinome, Neuroblastome, Mesotheliome, Nebennierenrindenkarzinome, Epithelkarzinome, Desmoidtumore, desmoplastische kleinzellige Rundzelltumore, endokrine Tumore, Tumore der Ewing-Sarkom-Familie, Keimzelltumore, Hepatoblastome, hepatozelluläre Karzinome, Lymphome, Melanome, Weichteilsarkome die keine Rhabdomyosarkome sind, Osteosarkome, periphere primitive neuroektodermale Tumore, Retinoblastome, Rhabdomyosarkome und Wilms-Tumore.

## Revendications

1. Composition pharmaceutique comprenant un peptide ciblant les intégrines α5β1 et αvβ3 pour utilisation dans le traitement du cancer chez un patient, dans laquelle :
(a) le peptide présente la séquence d'acides aminés de SEQ ID NO :1 à 6, ou est un dérivé de celui-ci qui est un peptide de SEQ ID NOs :7 à 31 ; et
(b) une quantité efficace dudit peptide est à administrer à un patient atteint d'un cancer pendant une à quatre semaines, suivi d'une thérapie par inhibiteur de point de contrôle immunitaire.

2. Composition pour l'utilisation selon la revendication 1, dans laquelle le peptide est administré au moins une fois par jour pendant une à quatre semaines.

3. Composition pour l'utilisation selon la revendication 1, dans laquelle la thérapie par inhibiteur de point de contrôle immunitaire est un agent qui inhibe une interaction entre PD-1 et PD-L1 ; ou entre CTLA-4 et B7.

4. Composition pour l'utilisation selon la revendication 1, dans laquelle l'inhibiteur de point de contrôle immunitaire cible LAG-3, KIR, OX40L, IDO-1 et TIM-3.

5. Composition pour l'utilisation selon la revendication 1, dans laquelle le patient est non réactif ou seulement partiellement réactif à l'inhibiteur de point de contrôle immunitaire seul.

6. Composition pour l'utilisation selon la revendication 1, dans laquelle le patient a un cancer de stade précoce, ou un cancer de stade avancé, ou dans laquelle le cancer est non résécable.

7. Composition pour l'utilisation selon la revendication 1, dans laquelle le cancer est un sarcome, un carcinome ou un cancer à tumeur solide sélectionné parmi les tumeurs de lignée germinale, les tumeurs du système nerveux central, le cancer du sein, le cancer de la prostate, le cancer du col de l'utérus, le cancer de l'utérus, le cancer du poumon, le cancer de l'ovaire, le cancer testiculaire, le cancer de la thyroïde, un astrocytome, un gliome, le cancer pancréatique, le cancer de l'estomac, le cancer du foie, le cancer du côlon, un mélanome, le cancer du rein, le cancer de la vessie, le cancer de l'œsophage, le cancer du larynx, le cancer de la parotide, le cancer du tractus biliaire, le cancer rectal, le cancer de l'endomètre, des carcinomes à cellules squameuses, des adénocarcinomes, des carcinomes à petites cellules, des neuroblastomes, des mésothéliomes, des carcinomes corticosurrénaux, des carcinomes épithéliaux, des tumeurs desmoïdes, des tumeurs à petites cellules rondes desmoplastiques, des tumeurs endocrines, des tumeurs de la famille des sarcomes d'Ewing, des tumeurs de cellules germinales, des hépatoblastomes, des carcinomes hépatocellulaires, des lymphomes, des mélanomes, des sarcomes des tissus mous de non-rhadbomyosarcome, des ostéosarcomes, des tumeurs neuroectodermiques primitives périphériques, des rétinoblastomes, des rhadbomyosarcomes et des tumeurs de Wilms.
